# DEMANDE DE BREVET EUROPEEN

(11) **EP 2 710 992 A2**
(43) Date de publication de la demande: **26.03.2014**
(21) Numéro de dépôt: 13185838.3
(22) Date de dépôt: 24.09.2013
(51) Int. Cl.: A61J 1/10, B65B 55/02, B65B 55/06, B65B 55/14, B65D 81/02, B65B 3/00

(54) **Méthode de fabrication et remplissage d'une poche souple préremplie d'une solution à perfuser destinée à la perfusion ambulatoire**

(30) Priorité: 25.09.2012 FR 1258993
(71) Demandeur: Pharmadyne, 75009 Paris (FR)
(72) Inventeur: Rosoor, Lucas, 94130 NOGENT SUR MARNE (FR); Malfait, Benjamin, 75013 PARIS (FR)
(74) Mandataire: Cabinet Plasseraud

(57) **Abrégé**

La présente invention concerne l'utilisation d'une poche souple préremplie d'une solution dans un système de vidage non gravitationnel, notamment pour l'administration de ladite solution à l'aide d'une pompe à perfusion ambulatoire, ainsi qu'un procédé de vidage d'une poche souple préremplie d'une solution.

## Description

La présente invention concerne l'utilisation d'une poche souple préremplie d'une solution dans un système de vidage non gravitationnel et sur un procédé de vidage de ladite poche souple. En d'autres termes, la présente invention porte sur l'utilisation d'une poche souple préremplie d'une solution pour un écoulement non gravitationnel de la solution. Ainsi, l'utilisation selon l'invention est particulièrement avantageuse pour l'administration d'une solution à perfuser à l'aide d'une pompe à perfusion ambulatoire.

Les poches contenant des médicaments à perfuser sont, pour la plupart, utilisées par gravité, c'est-à-dire qu'elles sont accrochées à une patère par un oeillet situé à une extrémité de la poche. La solution contenue dans la poche s'écoule alors par gravité par un port opposé à l'oeillet dans une tubulure jusqu'au patient.

Les poches destinées à être utilisées en ambulatoire et perfusées grâce à une pompe à perfusion doivent pouvoir être utilisées quelque soit leur position. Si on utilise une poche classique destinée à être utilisée par gravité en ambulatoire, il y a un risque important d'occlusion. En effet, lorsque la poche se vide, les parties de la membrane en vis-à-vis peuvent adhérer l'une à l'autre et bloquer l'écoulement de la solution restant à perfuser vers l'orifice de sortie. Ce risque est d'autant plus important que les pompes ne détectent pas systématiquement ces occlusions. Or cela peut avoir des conséquences graves pouvant entrainer un risque vital. En effet, le patient peut ne pas recevoir la dose prescrite. Le médecin ne sait pas que le patient n'a pas reçu la dose prescrite. Or comme le patient ne réagit pas le médecin décide d'augmenter le dosage, et le patient reçoit un surdosage. Selon la solution perfusée, il peut y avoir des conséquences graves sur l'organisme du patient pouvant mener jusqu'à son décès. Les poches classiques, c'est-à-dire présentant une surface interne lisse, ne sont par conséquent pas autorisées en perfusion ambulatoire, qu'elles soient préremplies, prêtes à l'emploi ou destinées à être remplies au moment de l'installation du système de perfusion.

Afin d'éviter ces problèmes d'occlusion il a été proposé des poches souples présentant une surface interne texturée, par exemple une surface striée ou granulée, ce qui empêche les parties de la membrane en vis-à-vis d'adhérer entre elles. Les poches texturées présentent ainsi un avantage certain pour la perfusion en ambulatoire lorsque les poches sont remplies à la demande juste avant la mise en place du système de perfusion.

Cependant, les poches à surface interne texturée ne sont pas autorisées pour la fabrication de poches préremplies prêtes à l'emploi destinées à la perfusion. En effet, la surface de contact entre la solution médicamenteuse et la membrane est beaucoup plus importante pour une poche à surface interne texturée que pour une poche à surface interne lisse. Or, du fait de la migration de certains composants de la membrane vers la solution médicamenteuse ou inversement, cette surface de contact accrue pose des problèmes de stabilité dans le cas de poches préremplies pouvant être stockées plusieurs mois. De plus, les poches à surface interne texturée ne présentent pas une transparence suffisante permettant un contrôle visuel de la solution à perfuser. Or, un contrôle visuel de la solution contenue dans une poche préremplie à perfuser est essentiel avant la mise en place du système de perfusion. Ainsi, il existe un besoin de proposer des poches préremplies prêtes à l'emploi pouvant être utilisées en perfusion ambulatoire sans risque d'occlusion.

La demanderesse a remarqué que, contre toute attente, une poche préremplie à surface interne lisse ayant subi un traitement thermique pouvait être utilisée en perfusion ambulatoire sans risque d'occlusion.

La présente invention porte par conséquent sur l'utilisation d'une poche souple préremplie d'une solution dans un système de vidage non gravitationnel,
la poche souple préremplie étant obtenue par un procédé comprenant :
- la fourniture d'une poche souple ;
- le remplissage de la poche avec ladite solution pour obtenir une poche souple préremplie ; et
- le traitement thermique de la poche souple préremplie.

En particulier, l'utilisation selon l'invention permet un écoulement non gravitationnel de la solution contenue dans la poche souple préremplie.

Le système de vidage non gravitationnel peut être une pompe à perfusion ambulatoire. Ainsi, dans un mode de réalisation particulier, l'utilisation selon l'invention est adaptée pour l'administration d'une solution à perfuser à l'aide d'une pompe à perfusion ambulatoire.

La présente invention ne porte cependant pas sur une méthode de traitement thérapeutique. En particulier, l'étape d'administration de la solution au patient est exclue de la portée de la présente invention.

Contrairement à une perfusion en milieu traditionnel où la poche contenant la solution à perfuser est suspendue à une potence et la solution à perfuser s'écoule par gravité, dans un système de perfusion ambulatoire, la solution à perfuser est aspirée par l'action mécanique de la pompe. Son contenu n'est donc pas libéré par écoulement gravitationnel. Or, le principe même de la perfusion ambulatoire fait que la poche peut se retrouver dans n'importe quelle position du fait des mouvements du patient. Par exemple, l'orifice de la poche peut se retrouver en position haute par rapport au reste de la poche ou encore la poche peut être pliée. La présente invention porte donc sur l'utilisation d'une poche souple, de préférence ladite poche souple pouvant être dans n'importe quelle position. Dans un mode de réalisation particulier, la poche souple n'est pas dans une position verticale avec l'orifice positionné vers le bas, de sorte que la solution qu'elle contient ne peut pas s'écouler par gravité.

La poche souple est constituée d'une membrane en matériau souple formant un volume intérieur. Elle présente de préférence une surface interne lisse. La poche comprend au moins un orifice permettant le remplissage et/ou le vidage de la poche. La membrane peut être constituée d'une gaine soudée, d'une feuille pliée et soudée ou d'un assemblage de plusieurs feuilles soudées entre elles, de préférence deux feuilles, de manière à délimiter un volume intérieur. Dans un mode de réalisation particulier la poche est formée deux feuilles identiques soudée entre elles sur leur pourtour. La surface non soudée présente de préférence une forme essentiellement rectangulaire ou ovoïde. La membrane est de préférence une membrane multicouche comprenant au moins une couche réalisée dans un matériau transparent empêchant toute migration de composés de l'intérieur de la poche vers l'extérieur ou inversement. La membrane comprend notamment une couche imperméable à l'oxygène. Des matériaux adaptés à la réalisation de la membrane sont, par exemple, les polyoléfines (polyéthylène, polypropylène, copolymères éthylène/propylène), les copolymères blocs styréniques (styrène/butadiène/styrène (SBS), styrène/éthylène/butylène/styrène (SEBS), styrène/isoprène/styrène (SIS), styrène/éthylène/propylène/styrène (SEPS)), les élastomères polyesters, le poly(chlorure de vinyle) (PVC) et le poly(éthylène-acétate de vinyle) (EVA). Une membrane adaptée à la présente invention est par exemple le film Nexcel™ M312 commercialisé par la société Sealed Air, New Jersey, USA. Une poche adaptée à l'utilisation selon l'invention est par exemple une poche SA multicouche réalisée avec un film Nexcel^{™} M312 et commercialisée par la société Sippex, Courzieux, France.

La poche souple est remplie d'une solution prête à l'emploi. La solution est de préférence une solution à perfuser prête à l'emploi, c'est-à-dire qu'elle est utilisable directement par simple connexion, via une tubulure adaptée, à une pompe à perfusion sans nécessiter de mélange ni de dilution préalable qui peuvent être source d'erreur. La solution à perfuser peut être toute solution à perfuser destinée à une administration par perfusion ambulatoire. La solution à perfuser est par exemple une solution médicamenteuse ou une solution nutritive. Typiquement, une solution nutritive à perfuser est une solution glucosée contenant éventuellement des acides aminés, des lipides notamment sous forme d'émulsion, des minéraux, des oligoéléments essentiels ou des vitamines. Une solution médicamenteuse à perfuser est typiquement une solution physiologique contenant une concentration prédéterminée de principe actif. La solution physiologique est par exemple une solution de NaCl diluée à 9 pour 1000 dans de l'eau distillée. Elle peut comprendre un ou plusieurs principes actifs selon la pathologie à traiter. Les principes actifs peuvent être par exemple des analgésique, des antalgiques, des antibiotiques, des anticancéreux, des anti-inflammatoires, des antiviraux, des antirétroviraux ou encore des hormones comme l'insuline. De préférence, la solution médicamenteuse comprend un analgésique, plus préférentiellement un analgésique de niveau III tel que défini dans la classification OMS des antalgiques, notamment la morphine, l'hydromorphine, l'oxycodone, le fentanyl, la buprénorphine, la nalbuphine et la pentazocine. Le remplissage de la poche est réalisé avec une solution à perfuser ayant un dosage prédéterminé en fonction de l'application envisagée. Elle est de préférence remplie au maximum de sa capacité. On obtient alors une poche souple préremplie. La poche étant destinée à une administration par perfusion, elle ne doit pas contenir d'air.

Après l'étape de remplissage, la poche souple préremplie subit un traitement thermique. Ce traitement s'effectue à une température supérieure à 100°C et inférieure à la température de fusion de la membrane de la poche. Avantageusement, la température du traitement thermique est choisie de manière à être entre 80% et 98%, de préférence entre 85 et 95% de la température de fusion de la membrane. Typiquement, la température du traitement thermique pourra être de 110 à 140°C, de préférence de 115 à 130°C. Dans le cas d'une membrane multicouche, on entend par température de fusion de la membrane la température de fusion d'au moins l'un des matériaux constituant la membrane multicouche. De préférence, on entend par température de fusion d'une membrane multicouche, la température de fusion du matériau de la membrane ayant la température de fusion la moins élevée. La durée du traitement thermique doit être suffisante pour permettre à la poche de se déformer. Typiquement, le traitement thermique est réalisé pendant 5 à 40 minutes, de préférence pendant 10 à 30 minutes. Le traitement thermique est de préférence réalisé dans un autoclave. Lors de ce traitement thermique, la poche préremplie, qui est mise sous contrainte du fait de la pression interne créée par la solution à perfuser, est chauffée à une température proche du point de fusion de la membrane. Celle-ci se déforme pour réduire les contraintes mécaniques auxquelles elle est soumise. Après le traitement thermique, la poche est refroidie, généralement à température ambiante, avant stockage. Après refroidissement, la poche conserve sa déformation. Cette déformation est en particulier visible sur la membrane de la poche qui présente une forme différente de celle d'origine. Lors du vidage d'une poche non traitée thermiquement, la poche se replie, les parties de la membrane en vis-à-vis se rapprochent l'une de l'autre et ont tendance à se coller l'une à l'autre, notamment aux environs de l'orifice de sortie. Au contraire, la déformation de la poche selon l'invention est telle que, notamment aux environs de l'orifice de sortie, la membrane présente une déformation qui empêche les parties de la membrane en vis-à-vis de se superposer et de coller l'une à l'autre lorsque la poche est vidée. Le risque d'occlusion est par conséquent fortement réduit.

La poche souple préremplie ayant subi un traitement thermique est destinée à être utilisée dans un système de vidage non gravitationnel, c'est-à-dire pour un écoulement non gravitationnel de la solution qu'elle contient sans risque d'occlusion. Elle est particulièrement adaptée pour l'administration d'un traitement par perfusion intraveineuse ou sous-cutanée à l'aide d'une pompe à perfusion ambulatoire sans risque d'occlusion. Par pompe à perfusion ambulatoire on entend une pompe permettant la mise en place d'un système de perfusion ambulatoire notamment en milieu non traditionnel (c'est-à-dire hors hôpitaux, cliniques ou centres de perfusions). Une telle pompe est particulièrement adaptée à une utilisation sans contrainte de mobilité. Des exemples de pompes à perfusion ambulatoires disponibles dans le commerce sont les pompes Rythmic^{™} commercialisées par la société Micrel, SYNCHROMED® commercialisées par la société Medtronic, GemStar® commercialisées par la société Hospira, BodyGuard commercialisées par la société Ceasarea Medical Electronics (CME) ou Cadd commercialisées par la société Smith Medical. Lors de la mise en place du système de perfusion ambulatoire, la poche préremplie selon l'invention est reliée à la pompe via une tubulure adaptée à cette dernière, et la tubulure est reliée au patient par un cathéter adapté au mode d'administration désiré (intraveineux ou sous-cutané).

L'utilisation selon l'invention est adaptée à tout traitement par perfusion intraveineuse ou sous-cutanée compatible avec un système de perfusion ambulatoire, notamment les chimiothérapies, les traitements antalgiques autocontrôlés (PCA : Patient Controlled Analgesia, ou ACP : Antalgie Contrôlée par le Patient) ou les traitements antibiotiques.

La présente invention porte également sur un procédé de vidage d'une poche souple préremplie d'une solution, la poche souple préremplie étant obtenue par un procédé comprenant :
- la fourniture d'une poche souple ;
- le remplissage de la poche avec ladite solution pour obtenir une poche souple préremplie ; et
- le traitement thermique de la poche souple préremplie ;
   dans lequel la poche est dans une position ne permettant pas l'écoulement gravitationnel de la solution.

La poche et sont procédé d'obtention sont tels que définis précédemment.

Plus particulièrement, le procédé selon l'invention comprend :
- l'ouverture de la poche souple préremplie ;
- la disposition de la poche dans une position ne permettant pas l'écoulement gravitationnel ;et
- le vidage de la poche.

Le vidage de la poche peut être effectué par n'importe quel système de vidage non gravitationnel, notamment une pompe à perfusion ambulation telle que décrite ci-dessus.

L'exemple suivant illustre davantage l'invention et son intérêt. Cet exemple n'est présenté que dans un but d'illustration et ne peut être considéré comme limitatif de l'invention.

### Exemple

Un lot de poches de 100ml, multicouches, réalisée à partir d'un film Nexcel^{™} M312 et commercialisée sous la dénomination SA par la société Sippex a été remplie avec 100ml d'une solution physiologique de NaCl diluée à 9 pour 1000 dans de l'eau distillée et bouchée avec un port minitulipe sécable.

Les poches selon l'invention ont subi un traitement thermique en autoclave à 120°C et pression constante pendant 20 minutes, suivi d'une phase de refroidissement et repos à température ambiante pendant plus de 48h.

Les poches témoin n'ont subi aucun traitement thermique.

Les poches ont été connectées à une pompe GemStar® commercialisée par la société Hospira avec une tubulure et un embout de perfusion Hospira adaptés à la pompe GemStar®.

La pompe a été programmée en mode perfusion et bolus comme suit :
- Concentration : 1mg/ml
- Débit : 5ml/h
- dose de charge : non
- dose bolus : 5ml
- interdiction entre les bolus : 5 min
- aucune limite par heure
- réservoir : 100ml
- détection d'air activée

De tests de simulation de perfusion ont été effectués sur des poches traitées thermiquement selon l'invention dans différentes positions. Pour comparaison, les mêmes tests ont été effectués sur des poches non traitées.

Les tests de simulation de perfusion, consistent à procéder à l'écoulement de la solution à l'aide de la pompe, la poche étant disposée dans une position prédéfinie. La poche reste dans la même position tout au long du test. Les positions P1 à P6 utilisées pour les tests sont les suivantes :
P1 : posée sur une table et pliée sur elle-même port de sortie vers le haut ;
P2 : posée à plat sur une table et écrasée sous le poids de la pompe ;
P3 : posée à plat sur une table ;
P4 : roulée sur elle-même et coincée dans un verre ;
P5 : suspendue verticalement port de sortie vers le bas (position de gravité classique) ;
P6 : suspendue verticalement port de sortie vers le haut.

Pour chaque position, l'écoulement de la solution est initié par un bolus. Le test débute systématiquement avec une poche pleine de 100ml. Le test s'arrête dés qu'une occlusion est signalée par la pompe à perfusion. Le volume délivré par la pompe est alors relevé en mesurant le poids de la solution délivrée.

Les résultats pour chaque position, obtenus à partir d'une moyenne sur au moins trois tests pour chacune des positions, sont rassemblés dans le tableau ci-dessous.

| Position | Poches témoin | | Poches selon l'invention | |
|---|---|---|---|---|
| | Volume délivré avant obstruction (%) | Ecart type | Volume délivré avant obstruction (%) | Ecart type |
| P1 | 89 | 2 | 95 | 1 |
| P2 | 80 | 3 | 96 | 1 |
| P3 | 93 | 1 | 95 | 0 |
| P4 | 82 | 3 | 94 | 0 |
| P5 | 90 | 2 | 95 | 0 |
| P6 | 64 | 2 | 94 | 1 |
| Moyenne | 83 | | 95 | |

Les essais ont démontré qu'il y avait un risque d'occlusion important lorsqu'une poche non traitée n'est pas utilisée en position gravitationnelle.

Si l'on fait une moyenne des 6 positions, auxquelles les poches non traitées thermiquement ont été soumises, une occlusion est apparue après écoulement de 83% de solution à perfuser. Les résultats sont très dispersés. En effet, des occlusions ont été constatées dès 61 % du volume total de solution délivré.

Il apparaît donc risqué d'utiliser une poche non traitée au delà de 61% du volume total de solution à perfuser car des occlusions peuvent survenir.

Si l'on fait une moyenne des 6 positions auxquelles les poches traitées thermiquement selon l'invention ont été soumises, une occlusion est apparue seulement après écoulement de 95% de solution à perfuser. Les résultats sont très peu dispersés. Seule une occlusion isolée après un écoulement de 92% de solution a été constatée.

Au vu de ces résultats, les poches traitées thermiquement selon l'invention peuvent être utilisées sans risque d'occlusion jusqu'à 92% du volume total de solution à perfuser contre 61% pour les poches non traitées.

## Revendications

1. Utilisation d'une poche souple préremplie d'une solution dans un système de vidage non gravitationnel,
la poche souple préremplie étant obtenue par un procédé comprenant :
- la fourniture d'une poche ;
- le remplissage de la poche avec ladite solution pour obtenir une poche souple préremplie ; et
- le traitement thermique de la poche souple préremplie.

2. Utilisation selon la revendication 1, **caractérisée en ce que** la poche souple présente une surface interne lisse.

3. Utilisation selon l'une quelconque des revendications 1 ou 2, **caractérisée en ce que** la poche est une poche constituée de deux feuilles identiques soudées sur leur pourtour.

4. Utilisation selon l'une quelconque des revendications 1 à 3, **caractérisée en ce que** le traitement thermique est réalisé dans un autoclave.

5. Utilisation selon l'une quelconque des revendications 1 à 4, **caractérisée en ce que** le traitement thermique est réalisé à une température de 110 à 140°C.

6. Utilisation selon l'une quelconque des revendications 1 à 5, **caractérisée en ce que** le traitement thermique est réalisé pendant 5 à 40 minutes.

7. Utilisation selon l'une quelconque des revendications 1 à 6, **caractérisée en ce que** ladite poche souple peut être dans n'importe quelle position.

8. Utilisation selon l'une quelconque des revendications 1 à 7, **caractérisée en ce que** la solution comprend un principe actif choisi parmi les analgésiques, les antalgiques, les antibiotiques, les anticancéreux, les anti-inflammatoires, les antiviraux, les antirétroviraux et les hormones.

9. Utilisation selon l'une quelconque des revendications 1 à 8, **caractérisée en ce que** la solution comprend un analgésique de niveau III.

10. Procédé de vidage d'une poche souple préremplie d'une solution, la poche souple préremplie étant obtenue par un procédé comprenant :
- la fourniture d'une poche souple ;
- le remplissage de la poche avec ladite solution pour obtenir une poche souple préremplie ; et
- le traitement thermique de la poche souple préremplie ;
dans lequel la poche est dans une position ne permettant pas l'écoulement gravitationnel de la solution.
